# EUROPEAN PATENT APPLICATION

(11) **EP 4 722 703 A1**
(43) Date of publication of application: **08.04.2026**
(21) Application number: 24810856.5
(22) Date of filing: 01.05.2024
(51) Int. Cl.: G01N 27/12, G01N 27/04

(54) **HYDROGEN SENSOR, HYDROGEN DETECTION SYSTEM, AND SHEET FOR HYDROGEN SENSORS**

(30) Priority: 24.05.2023 JP 2023085202
(71) Applicant: Dai Nippon Printing Co., Ltd., Tokyo 162-8001 (JP)
(72) Inventor: ITOH, Nobuyuki, Tokyo 162-8001 (JP); MATSUMOTO, Tsukasa, Tokyo 162-8001 (JP); YOSHINO, Nobuhiro, Tokyo 162-8001 (JP); MIYASHIRO, Kae, Tokyo 162-8001 (JP); ISODA, Daisuke, Tokyo 162-8001 (JP); MISHIMA, Satoru, Tokyo 162-8001 (JP); ARAKI, Noboru, Tokyo 162-8001 (JP)
(74) Representative: Müller-Boré & Partner Patentanwälte PartG mbB
(86) International application number: PCT/JP2024/016796
(87) International publication number: WO 2024/241848

(57) **Abstract**

The present disclosure provides a hydrogen sensor including: a substrate, a sensitive film that is disposed on a first surface of the substrate, and that includes a catalyst dissociating hydrogen molecules, and tungsten oxide, a control unit that detects a change in electrical resistance, a first bus electrode and a second bus electrode disposed on the first surface of the substrate and connected to the control unit, a plurality of first sensor electrodes disposed in contact with the sensitive film on the first surface of the substrate and connected to the first bus electrode, and a plurality of second sensor electrodes disposed in contact with the sensitive film on the first surface of the substrate and connected to the second bus electrode, wherein the first sensor electrodes and the second sensor electrodes are alternately arranged at intervals in which a change in electrical resistance of the sensitive film can be detected.

## Description

### Technical Field

The present disclosure relates to a hydrogen sensor, a hydrogen detection system, and a sheet for hydrogen sensor.

### Background Art

In recent years, from the viewpoint of global environmental protection and prevention of fossil fuel depletion, clean and circulating energy is desired. In particular, research for using hydrogen gas as an energy source has been actively performed around the fuel cell. On the other hand, hydrogen gas has an explosion limit concentration of 4% to 75%, which is wide, and in order to widely use hydrogen gas as an energy source, handling such as storage and transportation of hydrogen and a safety device against hydrogen leakage are indispensable.

For example, although odor used in city gas is considered, in the case of hydrogen gas, problems such as poisoning of the fuel cell and deterioration of the gas turbine occur. For this reason, a safety measure in place of the odor is required. Then, in order to ensure safety, a hydrogen sensor that detects leakage of hydrogen gas is very important.

In the conventional hydrogen sensor, a contact combustion method or a semiconductor method is mainly used. In the contact combustion type hydrogen sensor, a catalyst metal such as platinum or palladium is heated by a heater, and hydrogen gas in contact with the catalyst is oxidized by oxygen in the air. The heat generated by the oxidation action of the hydrogen gas is electrically detected as a change in the conductivity of the catalyst metal. In addition, the semiconductor type hydrogen sensor detects a change in electrical characteristics of the sensitive film due to adsorption of the hydrogen gas to the sensitive film, that is, a change in electrical resistance. Similarly to the contact combustion type, the semiconductor type hydrogen sensor is used in a state of being heated by a heater. As described above, in the conventional hydrogen sensor such as the contact combustion method and the semiconductor method, since heating is performed, there has been a risk of the hydrogen gas that needs a measure for explosion-proof.

Also, in recent years, a gas-chromic type hydrogen sensor has been attracting attention. The gas-chromic type hydrogen sensor includes a metal oxide such as tungsten trioxide in which a color changes due to adsorption of hydrogen, and a catalyst such as platinum that dissociates hydrogen gas into a hydrogen atom, and optically detects hydrogen gas. Since the metal oxide such as tungsten trioxide also changes the electrical characteristics by the adsorption of hydrogen, the gas-chromic type hydrogen sensor can also electrically detect the hydrogen gas. (See, for example, Patent Documents 1 and 2)

### Citation List

### Patent Documents

Patent Document 1: Japanese Patent (JP-B) No. 4496204
Patent Document 2: Japanese Patent Application Laid-Open (JP-A) No. 2002-328108

### Summary

### Technical Problem

A hydrogen sensor such as a conventional contact combustion method or a semiconductor method detects hydrogen gas at a place where the hydrogen sensor is installed. Therefore, the hydrogen gas cannot be detected only in a narrow range. In addition, it is also difficult to specify a hydrogen leakage spot.

The present disclosure has been made in view of the above circumstances, and a main object thereof is to provide a hydrogen sensor capable of detecting hydrogen gas even in a wide range and also capable of specifying a hydrogen leakage spot.

### Solution to Problem

One embodiment of the present disclosure provides a hydrogen sensor including: a substrate, a sensitive film that is disposed on a first surface of the substrate, and that includes a catalyst dissociating hydrogen molecules, and tungsten oxide, a control unit that detects a change in electrical resistance, a first bus electrode and a second bus electrode disposed on the first surface of the substrate and connected to the control unit, a plurality of first sensor electrodes disposed in contact with the sensitive film on the first surface of the substrate and connected to the first bus electrode, and a plurality of second sensor electrodes disposed in contact with the sensitive film on the first surface of the substrate and connected to the second bus electrode, wherein the first sensor electrodes and the second sensor electrodes are alternately arranged at intervals in which a change in electrical resistance of the sensitive film can be detected.

Another embodiment of the present disclosure provides a hydrogen detection system using the hydrogen sensor described above.

Another embodiment of the present disclosure provides a sheet for hydrogen sensor in a roll shape, the sheet for hydrogen sensor including: a substrate, a sensitive film that is disposed on a first surface of the substrate and that includes a catalyst dissociating hydrogen molecules, and tungsten oxide, a bus electrode disposed on the first surface of the substrate and disposed in a meandering line shape, a plurality of first sensor electrodes and a plurality of second sensor electrodes disposed in contact with the sensitive film on the first surface of the substrate, wherein the first sensor electrodes and the second sensor electrodes are alternately arranged at intervals in which a change in electrical resistance of the sensitive film can be detected.

### Advantageous Effects

The hydrogen sensor in the present disclosure exhibit effects such that it can detect hydrogen gas even in a wide range, and can also specify a hydrogen leakage spot.

### Brief Description of Drawings

[FIG. 1] is a schematic plan view illustrating an example of a hydrogen sensor in the present disclosure.
[FIG. 2] is a schematic plan view illustrating an example of a hydrogen sensor in the present disclosure.
[FIG. 3] is a schematic plan view illustrating an example of a hydrogen sensor in the present disclosure.
[FIG. 4] is a schematic plan view illustrating an example of a hydrogen sensor in the present disclosure.
[FIG. 5] is a schematic plan view illustrating an example of a hydrogen sensor in the present disclosure.
[FIG. 6] is a schematic plan view illustrating an example of a hydrogen sensor in the present disclosure.
[FIG. 7] is a schematic plan view illustrating an example of a hydrogen sensor in the present disclosure.
[FIG. 8] is a schematic plan view illustrating an example of a hydrogen sensor in the present disclosure.
[FIG. 9] is a schematic plan view illustrating an example of a hydrogen sensor in the present disclosure.
[FIG. 10] is a schematic plan view illustrating an example of a hydrogen sensor in the present disclosure.
[FIG. 11] is a schematic plan view illustrating an example of a hydrogen sensor in the present disclosure.
[FIG. 12] is a schematic diagram illustrating an example of a hydrogen detection system in the present disclosure.
[FIG. 13] is a schematic diagram illustrating an example of a hydrogen detection system in the present disclosure.
[FIG. 14] is a schematic perspective view illustrating an example of a sheet for hydrogen sensor in the present disclosure.
[FIG. 15] is a schematic plan view illustrating an example of a sheet for hydrogen sensor in the present disclosure.

### Description of Embodiments

Embodiments of the present disclosure will be described below with reference to the drawings and the like. However, the present disclosure can be implemented in many different aspects, and is not to be construed as being limited to the description of the embodiments described below. In addition, in order to clarify the description, a width, a thickness, a shape, and the like of each member may be schematically represented as compared with the embodiment, but this is merely an example, and the interpretation of the present disclosure is not limited. In addition, in the present specification and the drawings, elements similar to those described above with reference to the preceding drawings are denoted by the same reference numerals, and detailed description thereof may be omitted as appropriate.

In the present specification, when expressing an aspect in which another member is disposed on a certain member, the expression "on the surface side" or "on a surface" includes both a case in which another member is disposed directly above or immediately below so as to be in contact with a certain member, and a case in which another member is disposed above or below a certain member interposing another member.

Further, in the present specification, terms such as "sheet", "film", and "plate" are not distinguished from each other on the basis of only differences in terms of names. For example, a "sheet" is used in a sense including a member such as a film or a plate.

Hereinafter, the hydrogen sensor, the hydrogen detection system, and the sheet for hydrogen sensor in the present disclosure will be described in detail.

### A. Hydrogen sensor

A hydrogen sensor in the present disclosure includes: a substrate, a sensitive film that is disposed on a first surface of the substrate, and that includes a catalyst dissociating hydrogen molecules, and tungsten oxide, a control unit that detects a change in electrical resistance, a first bus electrode and a second bus electrode disposed on the first surface of the substrate and connected to the control unit, a plurality of first sensor electrodes disposed in contact with the sensitive film on the first surface of the substrate and connected to the first bus electrode, and a plurality of second sensor electrodes disposed in contact with the sensitive film on the first surface of the substrate and connected to the second bus electrode, wherein the first sensor electrodes and the second sensor electrodes are alternately arranged at intervals in which a change in electrical resistance of the sensitive film can be detected.

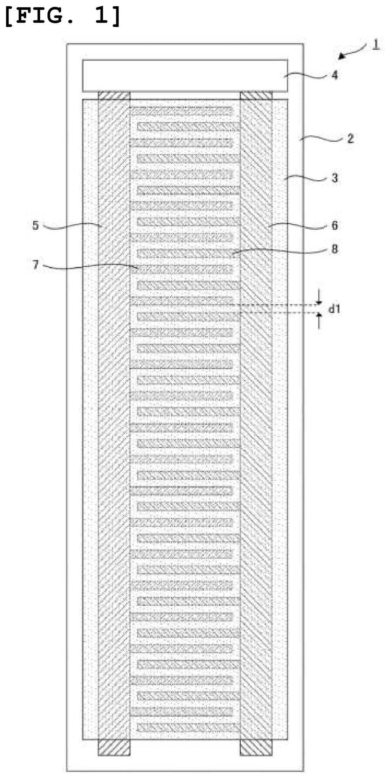

FIG. 1 is a schematic plan view illustrating an example of a hydrogen sensor in the present disclosure. As illustrated in FIG. 1, a hydrogen sensor 1 includes a substrate 2, a sensitive film 3 that is disposed on a first surface of the substrate 2 and that includes a catalyst dissociating hydrogen molecules, and tungsten oxide, a control unit 4 that detects a change in electrical resistance, a first bus electrode 5 and a second bus electrode 6 disposed on a first surface of the substrate 2 and connected to the control unit 4, a plurality of first sensor electrodes 7 disposed in contact with the sensitive film 3 on a first surface of the substrate 2 and connected to the first bus electrode 5, and a plurality of second sensor electrodes 8 disposed in contact with the sensitive film 3 on a first surface of the substrate 2 and connected to the second bus electrode 6. The first sensor electrodes 7 and the second sensor electrodes 8 are alternately arranged at an interval d1 capable of detecting a change in the electrical resistance of the sensitive film 3. In FIG. 1, the first sensor electrodes 7 and the second sensor electrodes 8 are a pair of comb-tooth electrodes.

The hydrogen sensor in the present disclosure is a hydrogen sensor using a decrease in electrical resistance when a sensitive film containing a catalyst and tungsten oxide reacts with hydrogen. The operation principle of the hydrogen sensor in the present disclosure will be described.

Tungsten oxide (WO₃) has a high electrical resistance. Therefore, in an atmosphere in which no hydrogen is present, the sensitive film has a high electrical resistance and an insulating property. At this time, the first sensor electrodes and the second sensor electrodes are insulated and are in a non-conductive state.

On the other hand, when the hydrogen molecules come into contact with the catalyst, the hydrogen molecules are dissociated and adsorbed to generate hydrogen atoms. These hydrogen atoms reduce tungsten oxide (WO₃) to produce an indefinite ratio compound (HₓWO₃ (0<x<1)). Since the indefinite ratio compound (HₓWO₃) is in the mixed valence state of W⁵⁺ and W⁶⁺, the electrical resistance is low. For this reason, in an atmosphere in which hydrogen is present, a reduction reaction of the tungsten oxide occurs, and the sensitive film has decreased electrical resistance and has conductivity. At this time, the first sensor electrodes and the second sensor electrodes are short-circuited to be in a conductive state.

Since the first bus electrode is connected to the first sensor electrodes, the second bus electrode is connected to the second sensor electrodes, and the control unit is connected to the first bus electrode and the second bus electrode, the hydrogen gas can be detected by detecting a change in the electrical resistance by the control unit.

In a conventional hydrogen sensor such as a semiconductor type or a contact combustion type, as described above, heating is required, and hydrogen gas can only be detected in a narrow range. On the other hand, the reduction reaction of the tungsten oxide does not require the supply of electricity. Thus, the hydrogen sensor in the present disclosure can have a large area, and can detect hydrogen gas in a relatively wide range at a low cost. Therefore, by using the hydrogen sensor in the present disclosure, it is possible to detect leakage of hydrogen gas not only in a small device such as a fuel cell but also in a large facility such as a hydrogen production facility, a hydrogen pipeline, a transport tanker, a storage tank, and a hydrogen power generation facility.

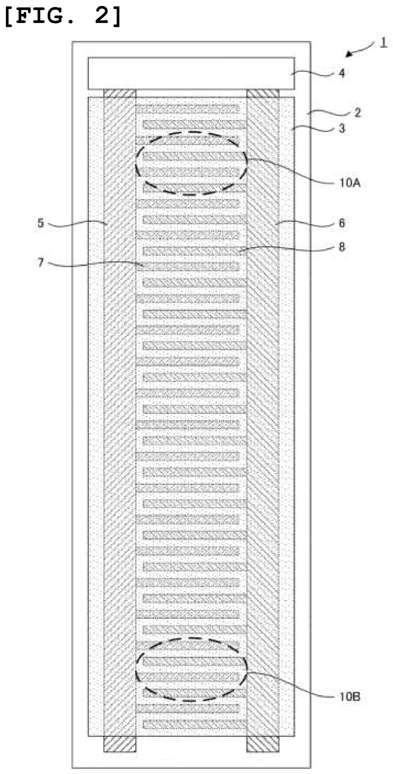

Also, the electrical resistance of the conductor is proportional to the length of the conductor. Thus, as illustrated in FIG. 2, when the reduction reaction of the tungsten oxide occurs in the region 10A, the electrical resistance of the sensitive film 3 decreases, and the first sensor electrodes 7 and the second sensor electrodes 8 are short-circuited, and when the reduction reaction of the tungsten oxide occurs in the region 10B, and the electrical resistance of the sensitive film 3 decreases and the first sensor electrodes 7 and the second sensor electrodes 8 are short-circuited, the values of the electrical resistance measured by the control unit 4 are different. For this reason, by measuring the electrical resistance by the control unit 4, it is possible to identify the position at which the electric resistance decreases due to the reaction of the sensitive film 3 with hydrogen. Therefore, by using the hydrogen sensor in the present disclosure, the hydrogen leakage spot can be identified.

In this manner, by using the hydrogen sensor in the present disclosure, hydrogen leakage can be detected even in a wide range at low cost, and a hydrogen leakage spot can be identified.

The hydrogen sensor in the present disclosure has three preferred embodiments. Hereinafter, each embodiment will be described.

### I. First embodiment

The first embodiment of the hydrogen sensor in the present disclosure is the hydrogen sensor described above, wherein one of the first bus electrode and one of the second bus electrode are each arranged in a line shape drawable with a one stroke, and one end of the first bus electrode and one end of the second bus electrode are connected to the control unit.

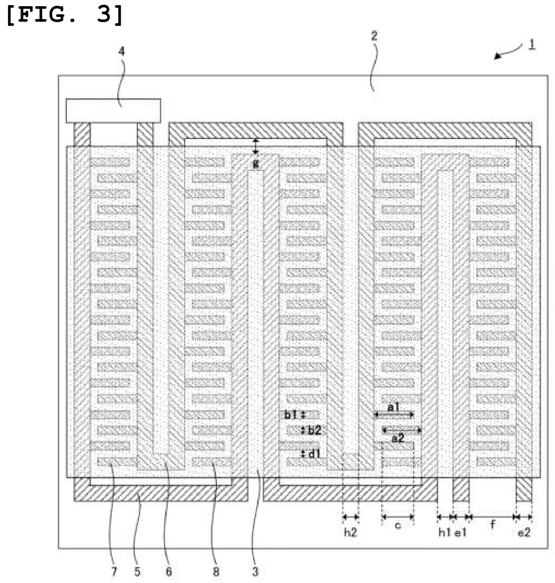

FIG. 3 is a schematic plan view illustrating an example of the hydrogen sensor of the present embodiment. As illustrated in FIG. 3, the hydrogen sensor 1 includes a substrate 2, a sensitive film 3 that is disposed on a first surface of the substrate 2 and that includes a catalyst dissociating hydrogen molecules, and tungsten oxide, a control unit 4 that detects a change in electrical resistance, one first bus electrode 5 and one second bus electrode 6 disposed on the first surface of the substrate 2 and connected to the control unit 4, a plurality of first sensor electrodes 7 disposed in contact with the sensitive film 3 on the first surface of the substrate 2 and connected to the first bus electrode 5, and a plurality of second sensor electrodes 8 disposed in contact with the sensitive film 3 on the first surface of the substrate 2 and connected to the second bus electrode 6. The first sensor electrodes 7 and the second sensor electrodes 8 are alternately arranged at intervals in which changes in the electrical resistance of the sensitive film 3 can be detected. In FIG. 3, the first sensor electrodes 7 and the second sensor electrodes 8 are a pair of comb-tooth electrodes. The first bus electrode 5 and the second bus electrode 6 are each arranged in a line shape drawable with a one stroke. In FIG. 3, the first bus electrode 5 and the second bus electrode 6 are arranged in a meandering line shape. In addition, one end of the first bus electrode 5 and one end of the second bus electrode 6 are connected to the control unit 4.

In the hydrogen sensor illustrated in FIG. 3, as described above, hydrogen gas can be detected by detecting a change in electrical resistance by the control unit. In addition, in the hydrogen sensor illustrated in FIG. 3, the control unit is one, and by measuring the electrical resistance by one control unit, the position at which the sensitive film reacts with hydrogen to decrease the electrical resistance can be identified.

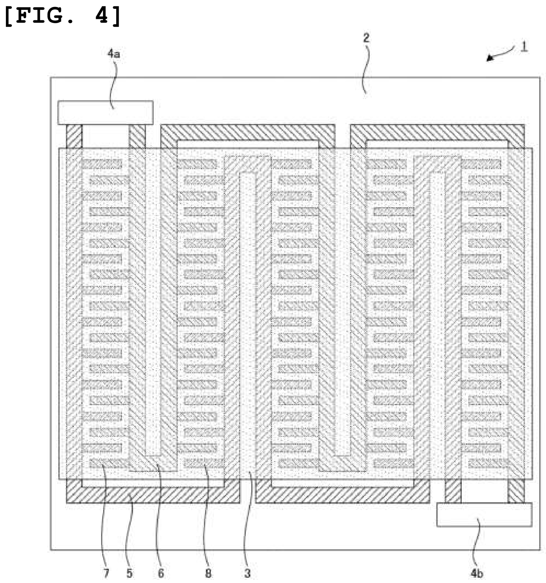

FIG. 4 is a schematic plan view illustrating another example of the hydrogen sensor of the present embodiment. The hydrogen sensor 1 illustrated in FIG. 4 is similar to the hydrogen sensor 1 illustrated in FIG. 3 except that the control unit includes a first control unit 4a connected to one end of the first bus electrode 5 and one end of the second bus electrode 6, and a second control unit 4b connected to the other end of the first bus electrode 5 and the other end of the second bus electrode 6.

In the hydrogen sensor illustrated in FIG. 4, as described above, hydrogen gas can be detected by detecting a change in electrical resistance by the control unit. In addition, in the hydrogen sensor illustrated in FIG. 4, the control unit includes two of the first control unit 4a and the second control unit 4b, so that, using the difference between the electrical resistance measured by the first control unit 4a and the electrical resistance measured by the second control unit 4b, the position at which the sensitive film 3 reacts with hydrogen to decrease the electrical resistance can be identified.

By using the hydrogen sensor of the present embodiment, as described above, hydrogen leakage can be detected even in a wide range at low cost, and further, a hydrogen leakage spot can be identified.

Hereinafter, the hydrogen sensor of the present embodiment will be described for each configuration.

### 1. First sensor electrodes and second sensor electrodes

In the present embodiment, a plurality of first sensor electrodes and a plurality of second sensor electrodes are disposed in contact with the sensitive film on the first surface of the substrate, and are alternately arranged at intervals in which a change in the electrical resistance of the sensitive film can be detected. The first sensor electrodes are connected to the first bus electrode, the second sensor electrodes are connected to the second bus electrode, and the first sensor electrodes and the second sensor electrodes are not connected.

In the present specification, the "interval in which the change in the electrical resistance of the sensitive film can be detected" refers to an interval in which the first sensor electrodes and the second sensor electrodes can be short-circuited when the reduction reaction of the tungsten oxide occurs and the electrical resistance of the sensitive film decreases.

The plurality of first sensor electrodes and the plurality of second sensor electrodes may be alternately arranged at intervals capable of detecting a change in electrical resistance of the sensitive film, and examples thereof include a pair of comb-tooth electrodes.

The interval between the first sensor electrodes and the second sensor electrodes may be an interval in which a change in the electrical resistance of the sensitive film can be detected. The interval may be, for example, 100 µm or more, and may be 500 µm or more. Also, the interval may be, for example, 10 mm or less, and may be 5 mm or less. That is, the interval may be, for example, 100 µm or more and 10 mm or less, and may be 500 µm or more and 5 mm or less.

The interval between the first sensor electrodes and the second sensor electrodes refers to the distance from the end portion of the first sensor electrode to the end portion of the second sensor electrode adjacent to each other. For example, in FIG. 3, the interval between the first sensor electrode and the second sensor electrode is indicated by d1.

The width of the first sensor electrode and the width of the second sensor electrode may be a width capable of detecting a change in the electrical resistance of the sensitive film. The width is, for example, 100 µm or more and may be 500 µm or more. Also, the width may be, for example, 10 mm or less, and may be 5 mm or less. That is, the width may be, for example, 100 µm or more and 10 mm or less, and may be 500 µm or more and 5 mm or less. For example, in FIG. 3, the width of the first sensor electrode is indicated by b1, and the width of the second sensor electrode is indicated by b2.

The length of the first sensor electrode and the length of the second sensor electrode may be a length capable of detecting a change in the electrical resistance of the sensitive film. The length is, for example, 10 mm or more and may be 50 mm or more. Also, the length may be, for example, 500 mm or less, and may be 100 mm or less. That is, the length may be, for example, 10 mm or more and 500 mm or less, and may be 50 mm or more and 100 mm or less. For example, in FIG. 3, the length of the first sensor electrode is indicated by a1, and the length of the second sensor electrode is indicated by a2.

The overlapping length of the first sensor electrode and the second sensor electrode may be a length capable of detecting a change in the electrical resistance of the sensitive film. The overlapping length is, for example, 9 mm or more and may be 45 mm or more. Also, the overlapping length may be, for example, 450 mm or less, and may be 90 mm or less. That is, the overlapping length may be, for example, 9 mm or more and 450 mm or less, and may be 45 mm or more and 90 mm or less. For example, in FIG. 3, the overlapping length of the first sensor electrode and the second sensor electrode is indicated by c.

The number of the first sensor electrodes and the number of the second sensor electrodes are appropriately set according to the size of the hydrogen sensor, the arrangement of the first bus electrode, the arrangement of the second bus electrode, and the like.

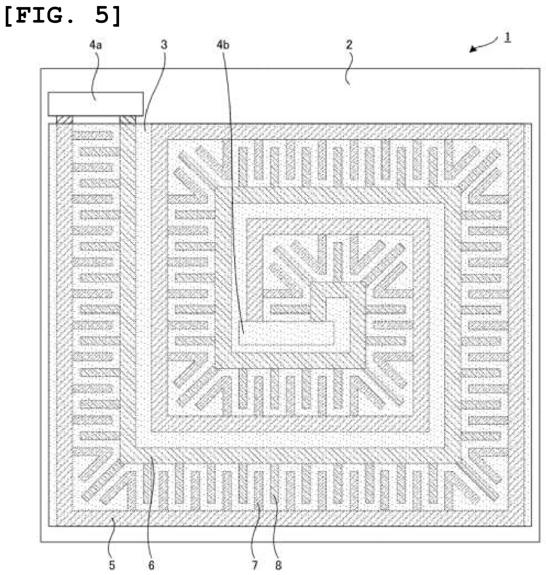

The shape of the first sensor electrode and the shape of the second sensor electrode are not particularly limited, and examples thereof include a linear shape, a polygonal line shape, and a curved line shape. For example, in FIG. 3, the shape of the first sensor electrode 7 and the shape of the second sensor electrode 8 are linear shape. In addition, for example, in FIG. 5, the shape of the first sensor electrode 7 is linear shape and polygonal shape, and the shape of the second sensor electrode 8 is linear shape.

Examples of the conductive material used for the first sensor electrodes and the second sensor electrodes include carbon and a metal material. Among them, carbon is preferable. Carbon is inert to hydrogen gas and is inexpensive. As will be described later, when the sensitive film, and the first sensor electrodes and the second sensor electrodes are arranged in this order on the first surface of the substrate, the conductive material used for the first sensor electrode and the second sensor electrode is preferably inert to the hydrogen gas. On the other hand, when the first sensor electrodes and the second sensor electrodes, and the sensitive film are arranged in this order on the first surface of the substrate, the conductive material used for the first sensor electrodes and the second sensor electrodes is not exposed to the hydrogen gas, and thus may be active or inert to the hydrogen gas.

The thickness of the first sensor electrode and the thickness of the second sensor electrode are not particularly limited as long as the thickness can function as an electrode, and is, for example, 0.1 µm or more and 2 µm or less.

The method of forming the first sensor electrode and the method of forming the second sensor electrode are not particularly limited, and include, for example, a method of forming and patterning a conductive film, a mask vapor deposition method, and a printing method. Examples of the method for forming the conductive film include a vacuum deposition method, a sputtering method, an ion plating method, and a plating method. Examples of the patterning method include an etching method and a lift-off method.

### 2. First bus electrode and second bus electrode

In the present embodiment, the first bus electrode and the second bus electrode are disposed on the first surface of the substrate and are connected to the control unit. The hydrogen sensor of the present embodiment includes one first bus electrode and one second bus electrode. The first bus electrode and the second bus electrode are arranged in a line shape drawable with a one stroke.

In the present specification, the term "line shape drawable with a one stroke" means that the line is configured by one continuous line and does not have a portion where a line overlaps.

The line shape drawable with a one stroke is not particularly limited as long as the first bus electrode, the second bus electrode, the first sensor electrodes, and the second sensor electrodes can be entirely disposed on the first surface of the substrate, and examples thereof include a meandering line shape as illustrated in FIG. 3 and FIG. 4, and a spiral shape as illustrated in FIG. 5. Among these, the first bus electrode and the second bus electrode are preferably arranged in a meandering line shape. In this case, since the first bus electrode and the second bus electrode can be formed by the roll-to-roll method, the hydrogen sensor can be efficiently mass-produced.

The first bus electrode and the second bus electrode are arranged so as to be able to specify a position at which the electrical resistance of the sensitive film has changed. Specifically, since the plurality of first sensor electrodes connected to the first bus electrode and the plurality of second sensor electrodes connected to the second bus electrode are alternately arranged, the first bus electrode and the second bus electrode are arranged along each other.

The width of the first bus electrode and the width of the second bus electrode may be a width that can function as an electrode. The width is, for example, 1 mm or more and may be 5 mm or more. Also, the width may be, for example, 50 mm or less, and may be 10 mm or less. That is, the width may be, for example, 1 mm or more and 50 mm or less, and may be 5 mm or more and 10 mm or less. For example, in FIG. 3, the width of the first bus electrode is indicated by e1, and the width of the second bus electrode is indicated by e2.

An interval between the first bus electrode and the second bus electrode facing each other across the first sensor electrodes and the second sensor electrodes may be an interval in which the first sensor electrodes and the second sensor electrodes can be disposed. The interval may be, for example, 11 mm or more, and may be 55 mm or more. Also, the interval may be, for example, 550 mm or less, and may be 110 mm or less. That is, the interval may be, for example, 11 mm or more and 550 mm or less, and may be 55 mm or more and 110 mm or less. For example, in FIG. 3, an interval between the first bus electrode and the second bus electrode facing each other across the first sensor electrodes and the second sensor electrodes is indicated by f.

The interval between the first bus electrode and the second bus electrode facing each other without sandwiching the first sensor electrodes and the second sensor electrodes may be an interval in which a change in the electrical resistance of the sensitive film cannot be detected. The interval may be, for example, 10 mm or more and may be 50 mm or more. Also, the interval may be, for example, 100 mm or less, and may be 70 mm or less. That is, the interval may be, for example, 10 mm or more and 100 mm or less, and may be 50 mm or more and 70 mm or less. When the interval is too small, the reduction reaction of the tungsten oxide occurs, and when the electrical resistance of the sensitive film decreases, the first bus electrode and the second bus electrode facing each other without sandwiching the first sensor electrodes and the second sensor electrodes are likely to be conductive, and it may be difficult to identify the hydrogen leakage spot. For example, in FIG. 3, the interval between the first bus electrode and the second bus electrode facing each other without sandwiching the first sensor electrodes and the second sensor electrodes is indicated by g.

The interval between the adjacent first bus electrodes and the interval between the adjacent second bus electrodes may be an interval in which a change in the electrical resistance of the sensitive film cannot be detected. The interval may be, for example, 10 mm or more and may be 50 mm or more. Also, the interval may be, for example, 100 mm or less, and may be 70 mm or less. That is, the interval may be, for example, 10 mm or more and 100 mm or less, and may be 50 mm or more and 70 mm or less. When the interval is too small, when the reduction reaction of the tungsten oxide occurs, and the electric resistance of the sensitive film decreases, the adjacent first bus electrodes or the adjacent second bus electrodes are likely to be electrically connected to each other, which may make it difficult to identify the hydrogen leakage spot. For example, in FIG. 3, an interval between adjacent first bus electrodes is indicated by h1, and an interval between adjacent second bus electrodes is indicated by h2.

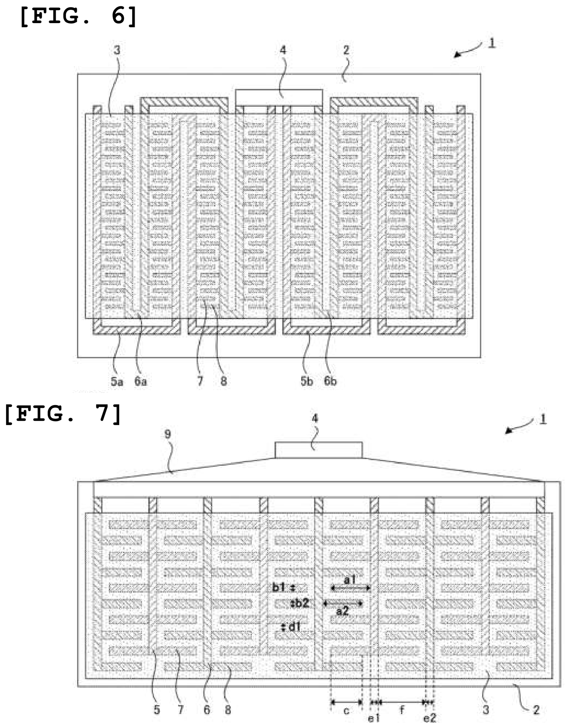

The first bus electrode and the second bus electrode are a pair of electrodes, and the number of the first bus electrodes and the number of the second bus electrodes are usually one. If the first bus electrode and the second bus electrode are a pair of electrodes, the number of the first bus electrodes and the number of the second bus electrodes may be two. For example, in FIG. 6, the hydrogen sensor 1 includes two first bus electrodes 5a and 5b and two second bus electrodes 6a and 6b, the first bus electrode 5a and the second bus electrode 6a serve as a pair of electrodes, and the first bus electrode 5b and the second bus electrode 6b serve as a pair of electrodes.

The conductive material used for the first bus electrode and the second bus electrode is the same as the conductive material used for the first sensor electrodes and the second sensor electrodes.

The thickness of the first bus electrode and the thickness of the second bus electrode are the same as the thickness of the first sensor electrode and the thickness of the second sensor electrode.

The method for forming the first bus electrode and the method for forming the second bus electrode are the same as the method for forming the first sensor electrode and the method for forming the second sensor electrode described above.

### 3. Sensitive film

The sensitive film in the present embodiment includes a catalyst dissociating hydrogen molecules, and tungsten oxide.

The sensitive film may be a single layer containing a catalyst and tungsten oxide, and may include, in order from the substrate side, a tungsten oxide layer containing tungsten oxide, and a catalyst layer containing a catalyst.

When the sensitive film includes a tungsten oxide layer and a catalyst layer, the catalyst layer may be a continuous film or a discontinuous film.

The catalyst is not particularly limited as long as it can dissociate hydrogen molecules into hydrogen ions (protons), and examples thereof include noble metals such as palladium, platinum, and iridium. The catalyst may be used in one kind alone or may be used in combination of two kinds or more.

Tungsten oxide is tungsten trioxide (WO₃).

The sensitive film may be disposed in contact with the first sensor electrodes and the second sensor electrodes, and the position of the sensitive film is not particularly limited. For example, the first sensor electrodes and the second sensor electrodes, and the sensitive film may be arranged in this order on the first surface of the substrate, and the sensitive film, and the first sensor electrodes and the second sensor electrodes may be arranged in this order on the first surface of the substrate.

When the sensitive film is a single layer containing a catalyst and tungsten oxide, the thickness of the sensitive film is not particularly limited as long as it can detect a change in the electrical resistance of the sensitive film, and is, for example, 100 nm or more and 3000 nm or less.

On the other hand, when the sensitive film includes a tungsten oxide layer and a catalyst layer, the thickness of the tungsten oxide layer is not particularly limited as long as it is a thickness capable of detecting a change in the electrical resistance of the tungsten oxide layer, and is, for example, 100 nm or more and 3000 nm or less. Also, the thickness of the catalyst layer is, for example, 1 nm or more and 10 nm or less.

When the sensitive film is a single layer containing a catalyst and tungsten oxide, examples of the method for forming the sensitive film include a sol-gel method. A method for forming a sensitive film by a sol-gel method can be referred to, for example, JP-B No. 5152797 and JP-B No. 5540248.

On the other hand, when the sensitive film includes a tungsten oxide layer and a catalyst layer, the method for forming the tungsten oxide layer is not particularly limited, and examples thereof include a vacuum deposition method, a sputtering method, and an ion plating method. The method for forming the catalyst layer is also not particularly limited, and examples thereof include a vacuum deposition method, a sputtering method, and an ion plating method.

### 4. Control unit

The control unit in the present embodiment is a member that detects a change in electrical resistance. The first bus electrode and the second bus electrode are connected to the control unit.

The control unit may be any control unit that can detect a change in electrical resistance, and examples thereof include those having an IC chip that detects a change in electrical resistance. As the IC chip, an open short type IC chip can be used. The open short type is an IC chip that detects a change in electrical resistance when the inter-terminal resistance becomes equal to or less than a set value, the flag becomes "1", and when the inter-terminal resistance becomes equal to or higher than another set value, it becomes a flag "0". Further, a capacitance type IC chip may be used as the IC chip. The capacitance type is typically an IC chip in which an antenna is detuned when a water droplet or the like comes into contact with the antenna, and the impedance change is detected. In the present disclosure, when a capacitance type IC chip is used, the sensitive film is separately disposed in contact with an antenna of an IC tag described later, and the impedance change is detected. Among them, an open short type IC chip is preferable. Examples of the open short type IC chip include UCODE G2iM+ from NXP Semiconductors.

Specifically, an IC tag including the IC chip and the antenna is used as the control unit. The IC tag is also referred to as an RF tag, an RFID tag, an electronic tag, a wireless tag, or the like. The IC tag may be the one including the IC chip and the antenna, and a general IC tag can be used. In the case of the IC tag, hydrogen gas can be detected using RFID.

The IC tag includes an active type that incorporates a power source (battery) and a passive type that does not incorporate a power source (battery). In particular, a passive tag is preferable because it works by obtaining a driving power source by receiving a radio wave supplied from the outside with an antenna, but not mounting a power supply (battery) on itself.

An example of the operation principle of the hydrogen sensor in the present disclosure when the control unit is an IC tag will be described. As described above, in an atmosphere in which no hydrogen is present, the sensitive film has a high electrical resistance and an insulating property. At this time, the first sensor electrodes and the second sensor electrodes are insulated and are in a non-conductive state. Thus, even when power is supplied from the external device to the IC tag, no current flows through the first sensor electrodes and the second sensor electrodes via the sensitive film. On the other hand, in an atmosphere in which hydrogen is present, a reduction reaction of the tungsten oxide occurs, and the sensitive film decreases in electrical resistance and has conductivity. At this time, the first sensor electrodes and the second sensor electrodes are short-circuited to be in a conductive state. Thus, when power is supplied from the external device to the IC tag, a current flows through the sensitive film to the first sensor electrodes and the second sensor electrodes. In the IC chip, the value of the electrical resistance is measured, information is detected from the value of the electrical resistance, and a signal corresponding to the information is transmitted to the external device. In the external device, hydrogen leakage is detected on the basis of the information, and a hydrogen leakage spot is identified.

The control unit is at least one. The control unit may be one, and may include two of the first control unit and the second control unit. When the control unit is one, one end of the first bus electrode and one end of the second bus electrode are connected to one control unit. When the control unit includes two of the first control unit and the second control unit, one end of the first bus electrode and one end of the second bus electrode are connected to the first control unit, and the other end of the first bus electrode and the other end of the second bus electrode are connected to the second control unit.

Among them, the control unit preferably includes two of the first control unit and the second control unit. As described above, when the control unit is connected to both ends of the first bus electrode and both ends of the second bus electrode, using the difference between the electrical resistance measured by the first control unit and the electrical resistance measured by the second control unit, the position at which the sensitive film reacts with hydrogen to decrease the electrical resistance can be identified. Thereby, it is possible to suppress an increase in electrical resistance due to the length of the first bus electrode and the length of the second bus electrode, and to reduce the electrical load of the control unit. Therefore, the reliability can be further enhanced, and the accuracy of the hydrogen sensor can be improved.

### 5. Substrate

The substrate in the present embodiment is a member that supports the sensitive film, the first sensor electrodes, the second sensor electrodes, the first bus electrode, and the second bus electrode, and has insulating properties.

The substrate is not particularly limited as long as it has insulating properties, and examples thereof include a glass substrate, a resin substrate, a ceramic substrate, and a silicon substrate including an insulating film on the surface.

The thickness of the substrate is not particularly limited, and is, for example, 10 µm or more and 2 mm or less.

### 6. Other configurations

In the present embodiment, the sensitive film, the first sensor electrodes, the second sensor electrodes, the first bus electrode, and the second bus electrode may be disposed on the first surface of the substrate. The sensitive film, the first sensor electrodes, the second sensor electrodes, the first bus electrode, and the second bus electrode may be disposed on only one surface of the substrate, or the sensitive film, the first sensor electrodes, the second sensor electrodes, the first bus electrode, and the second bus electrode may be disposed on both surfaces of the substrate, respectively.

In the hydrogen sensor of the present embodiment, it is possible to increase the area by appropriately designing the arrangement of the first bus electrode and the second bus electrode. The size of the hydrogen sensor of the present embodiment is not particularly limited, and for example, the width is 0.3 m or more and 2 m or less, and the length is 0.3 m or more and 10 m or less.

### II. Second embodiment

One embodiment of a hydrogen sensor in the present disclosure is the hydrogen sensor described above, wherein a plurality of the first bus electrode and a plurality of the second bus electrode are connected to the control unit via a flexible printed circuit board.

FIG. 7 is a schematic plan view illustrating an example of the hydrogen sensor of the present embodiment. As illustrated in FIG. 7, the hydrogen sensor 1 includes a substrate 2, a sensitive film 3 that is disposed on a first surface of the substrate 2 and includes a catalyst dissociating hydrogen gas into hydrogen atoms, and tungsten oxide, a control unit 4 that detects a change in electrical resistance, a plurality of first bus electrodes 5 and a plurality of second bus electrodes 6 disposed on the first surface of the substrate 2 and connected to the control unit 4, a plurality of first sensor electrodes 7 disposed in contact with the sensitive film 3 on the first surface of the substrate 2 and connected to the first bus electrode 5, and a plurality of second sensor electrodes 8 disposed in contact with the sensitive film 3 on the first surface of the substrate 2 and connected to the second bus electrode 6. The first sensor electrode 7 and the second sensor electrode 8 are alternately arranged at intervals in which changes in the electrical resistance of the sensitive film 3 can be detected. In FIG. 7, the first sensor electrodes 7 and the second sensor electrodes 8 are a pair of comb-tooth electrodes. The plurality of first bus electrodes 5 and the plurality of second bus electrodes 6 are connected to the control unit 4 via the flexible printed circuit board 9.

In the hydrogen sensor illustrated in FIG. 7, as described above, hydrogen gas can be detected by detecting a change in electrical resistance by the control unit. In addition, in the hydrogen sensor illustrated in FIG. 7, the control unit is one, and by measuring the electrical resistance by one control unit, the position at which the sensitive film reacts with hydrogen to decrease the electrical resistance can be identified.

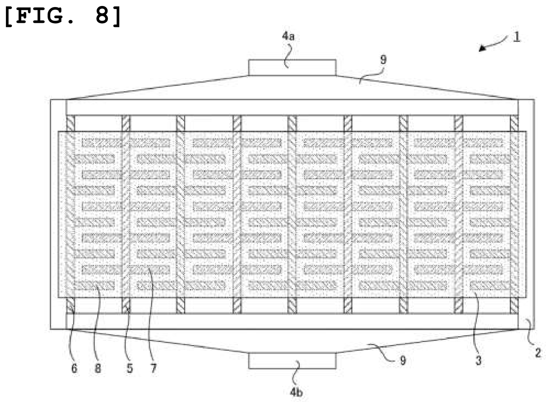

FIG. 8 is a schematic plan view showing another example of the hydrogen sensor of the present embodiment. The hydrogen sensor 1 shown in FIG. 8 is similar to the hydrogen sensor 1 shown in FIG. 7 except that the control unit includes a first control unit 4a connected to one end of the first bus electrode 5 and one end of the second bus electrode 6, and a second control unit 4b connected to the other end of the first bus electrode 5 and the other end of the second bus electrode 6.

In the hydrogen sensor illustrated in FIG. 8, as described above, hydrogen gas can be detected by detecting a change in electrical resistance by the control unit. In addition, in the hydrogen sensor illustrated in FIG. 8, the control unit has two of the first control unit 4a and the second control unit 4b, and using the difference between the electrical resistance measured by the first control unit 4a and the electrical resistance measured by the second control unit 4b, the position at which the sensitive film 3 reacts with hydrogen to decrease the electrical resistance can be identified.

By using the hydrogen sensor of the present embodiment, as described above, hydrogen leakage can be detected even in a wide range at low cost, and a hydrogen leakage spot can be identified. Further, in the present embodiment, since the lengths of the first bus electrode and the second bus electrode can be shortened as compared with the first embodiment, it is possible to suppress an increase in electrical resistance due to the length of the first bus electrode and the length of the second bus electrode. Therefore, the accuracy of the hydrogen sensor can be improved.

In addition, in the present embodiment, since the first bus electrodes and the second bus electrodes can be linearly arranged, the risk of disconnection can be reduced.

Hereinafter, the hydrogen sensor of the present embodiment will be described for each configuration.

### 1. First sensor electrode and second sensor electrode

In the present embodiment, a plurality of first sensor electrodes and a plurality of second sensor electrodes are disposed in contact with the sensitive film on the first surface of the substrate, and are alternately arranged at intervals in which a change in the electrical resistance of the sensitive film can be detected. The first sensor electrodes are connected to the first bus electrode, the second sensor electrodes are connected to the second bus electrode, and the first sensor electrodes and the second sensor electrodes are not connected.

The interval between the first sensor electrodes and the second sensor electrodes may be an interval in which a change in the electrical resistance of the sensitive film can be detected. The interval may be, for example, 100 µm or more, and may be 500 µm or more. Also, the interval may be, for example, 10 mm or less, and may be 5 mm or less. That is, the interval may be, for example, 100 µm or more and 10 mm or less, and may be 500 µm or more and 5 mm or less.

The interval between the first sensor electrodes and the second sensor electrodes refers to the distance from the end portion of the first sensor electrode to the end portion of the second sensor electrode adjacent to each other. For example, in FIG. 7, the interval between the first sensor electrode and the second sensor electrode is indicated by d1.

The width of the first sensor electrode and the width of the second sensor electrode may be a width capable of detecting a change in the electrical resistance of the sensitive film. The width is, for example, 100 µm or more and may be 500 µm or more. Also, the width may be, for example, 10 mm or less, and may be 5 mm or less. That is, the width may be, for example, 100 µm or more and 10 mm or less, and may be 500 µm or more and 5 mm or less. For example, in FIG. 7, the width of the first sensor electrode is indicated by b1, and the width of the second sensor electrode is indicated by b2.

The length of the first sensor electrode and the length of the second sensor electrode may be a length capable of detecting a change in the electrical resistance of the sensitive film. The length is, for example, 10 mm or more and may be 50 mm or more. Also, the length may be, for example, 500 mm or less, and may be 100 mm or less. That is, the length may be, for example, 10 mm or more and 500 mm or less, and may be 50 mm or more and 100 mm or less. For example, in FIG. 7, the length of the first sensor electrode is indicated by a1, and the length of the second sensor electrode is indicated by a2.

The overlapping length of the first sensor electrode and the second sensor electrode may be any length capable of detecting a change in the electrical resistance of the sensitive film. The overlapping length is, for example, 9 mm or more and may be 45 mm or more. Also, the overlapping length may be, for example, 450 mm or less, and may be 90 mm or less. That is, the overlapping length may be, for example, 9 mm or more and 450 mm or less, and may be 45 mm or more and 90 mm or less. For example, in FIG. 7, the overlapping length of the first sensor electrode and the second sensor electrode is indicated by c.

The number of the first sensor electrodes and the number of the second sensor electrodes are appropriately set according to the size of the hydrogen sensor, the arrangement of the first bus electrode, the arrangement of the second bus electrode, and the like.

The shape of the first sensor electrode and the shape of the second sensor electrode are not particularly limited, and examples thereof include a linear shape, a polygonal line shape, and a curved line shape.

The conductive material used for the first sensor electrodes and the second sensor electrodes, the thickness of the first sensor electrode and the second sensor electrode, and the method for forming the first sensor electrode and the second sensor electrode are the same as those of the first sensor electrodes and the second sensor electrodes in the first embodiment.

### 2. First bus electrode and second bus electrode

In the present embodiment, the first bus electrode and the second bus electrode are disposed on the first surface of the substrate and are connected to the control unit. The hydrogen sensor of the present embodiment includes a plurality of first bus electrodes and a plurality of second bus electrodes.

The first bus electrodes and the second bus electrodes are a pair of electrodes and are alternately arranged.

The width of the first bus electrode and the width of the second bus electrode may be a width that can function as an electrode. The width is, for example, 1 mm or more and may be 5 mm or more. Also, the width may be, for example, 50 mm or less, and may be 10 mm or less. That is, the width may be, for example, 1 mm or more and 50 mm or less, and may be 5 mm or more and 10 mm or less. For example, in FIG. 7, the width of the first bus electrode is indicated by e1, and the width of the second bus electrode is indicated by e2.

The interval between the first bus electrode and the second bus electrode may be an interval in which the first sensor electrodes and the second sensor electrodes can be disposed. The interval may be, for example, 11 mm or more, and may be 55 mm or more. Also, the interval may be, for example, 550 mm or less, and may be 110 mm or less. That is, the interval may be, for example, 11 mm or more and 550 mm or less, and may be 55 mm or more and 110 mm or less. For example, in FIG. 7, the interval between the first bus electrode and the second bus electrode is indicated by f.

The number of the first bus electrodes and the number of the second bus electrodes are plural. The first bus electrodes and the second bus electrodes may be a pair of electrodes, and the number of the first bus electrodes and the number of the second bus electrodes may be the same or different. For example, in FIG. 7, four first bus electrodes 5 and five second bus electrodes 6 are provided, and the number of the first bus electrodes and the number of the second bus electrodes are different.

The conductive material used for the first bus electrode and the second bus electrode is the same as the conductive material used for the first sensor electrodes and the second sensor electrodes.

The thickness of the first bus electrode and the thickness of the second bus electrode are the same as the thickness of the first sensor electrode and the thickness of the second sensor electrode.

The method for forming the first bus electrodes and the method for forming the second bus electrodes are the same as the method for forming the first sensor electrodes and the method for forming the second sensor electrodes described above.

### 3. Sensitive film

The sensitive film in the present embodiment includes a catalyst that dissociates hydrogen gas into hydrogen atoms, and tungsten oxide. The sensitive film is the same as the sensitive film in the first embodiment.

### 4. Control unit

The control unit in the present embodiment is a member that detects a change in electrical resistance. The first bus electrode and the second bus electrode are connected to the control unit.

The control unit is the same as the control unit in the first embodiment.

The control unit is at least one. The control unit may be one, and may include two of the first control unit and the second control unit. When the control unit is one, one end of the first bus electrode and one end of the second bus electrode are connected to one control unit. When the control unit includes two of the first control unit and the second control unit, one end of the first bus electrode and one end of the second bus electrode are connected to the first control unit, and the other end of the first bus electrode and the other end of the second bus electrode are connected to the second control unit.

### 5. Flexible printed circuit board

In the present embodiment, a plurality of first bus electrodes and a plurality of second bus electrodes are connected to the control unit via a flexible printed circuit board (FPC). A general FPC can be used as the FPC.

### 6. Substrate

The substrate in the present embodiment is a member that supports the sensitive film, the first sensor electrodes, the second sensor electrodes, the first bus electrode, and the second bus electrode, and has insulating properties. The substrate is the same as the substrate in the first embodiment.

### III. Third embodiment

The third embodiment of the hydrogen sensor in the present disclosure is the hydrogen sensor described above, wherein a plurality of the first bus electrode are disposed along a first direction, a plurality of the second bus electrode are disposed along a second direction orthogonal to the first direction, the control unit includes a third control unit connected to one end of the plurality of the first bus electrode, and a fourth control unit connected to one end of the plurality of the second bus electrode, and an insulating film is disposed between the first bus electrode and the second bus electrode in a region where the first bus electrode and the second bus electrode intersect.

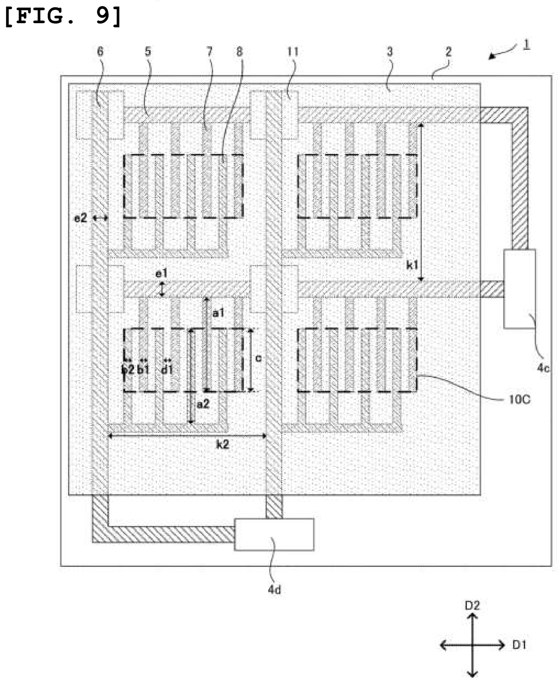

FIG. 9 is a schematic plan view illustrating an example of the hydrogen sensor of the present embodiment. As illustrated in FIG. 9, the hydrogen sensor 1 includes a substrate 2, a sensitive film 3 that is disposed on a first surface of the substrate 2 and includes a catalyst dissociating hydrogen molecules, and tungsten oxide, a third control unit 4c and a fourth control unit 4d that detects a change in electrical resistance, a plurality of second bus electrodes 6 disposed on the first surface of the substrate 2 and connected to a plurality of first bus electrodes 5 and the fourth control unit 4d connected to the third control unit 4c, a plurality of first sensor electrodes 7 disposed in contact with the sensitive film 3 on the first surface of the substrate 2 and connected to the first bus electrode 5, and a plurality of second sensor electrodes 8 disposed in contact with the sensitive film 3 on the first surface of the substrate 2 and connected to the second bus electrode 6. The first sensor electrodes 7 and the second sensor electrodes 8 are alternately arranged at intervals in which a change in the electrical resistance of the sensitive film 3 can be detected. In FIG. 9, the first sensor electrodes 7 and the second sensor electrodes 8 are a pair of comb-tooth electrodes. The plurality of first bus electrodes 5 are linearly arranged in the first direction D1, and the plurality of second bus electrodes 6 are linearly arranged in the second direction D2 orthogonal to the first direction D1. The control unit includes a third control unit 4c connected to one end of the plurality of first bus electrodes 5, and a fourth control unit 4d connected to one end of the plurality of second bus electrodes 6. In a region where the first bus electrodes 5 and the second bus electrodes 6 intersect, an insulating film 11 is disposed between the first bus electrodes 5 and the second bus electrodes 6.

In the hydrogen sensor illustrated in FIG. 9, as described above, hydrogen gas can be detected by detecting a change in electrical resistance by the control unit. In addition, in the hydrogen sensor illustrated in FIG. 9, by measuring the electrical resistance by the third control unit and the fourth control unit, it is possible to specify the intersection of the first bus electrodes and the second bus electrodes with reduced electrical resistance among the intersections of the first bus electrodes and the second bus electrodes. Therefore, the intersection of the first bus electrode and the second bus electrode with reduced electrical resistance can be identified as a position where the sensitive film reacts with hydrogen to decrease the electrical resistance.

By using the hydrogen sensor of the present embodiment, as described above, hydrogen leakage can be detected even in a wide range at low cost, and a hydrogen leakage spot can be further identified.

In addition, in the present embodiment, since it is not necessary to arrange the first bus electrodes and the second bus electrodes in a line shape drawable with a one stroke, the risk of disconnection can be reduced. Further, as shown in FIG. 9, since the area of the portion 10C where the first sensor electrodes 7 and the second sensor electrodes 8 overlap can be made constant, the variation of the signal is reduced, and the detection sensitivity is improved.

Hereinafter, the hydrogen sensor of the present embodiment will be described for each configuration.

### 1. First sensor electrode and second sensor electrode

In the present embodiment, the plurality of first sensor electrodes and the plurality of second sensor electrodes are disposed in contact with the sensitive film on the first surface of the substrate, and are alternately arranged at intervals in which a change in the electrical resistance of the sensitive film can be detected. The first sensor electrodes are connected to the first bus electrode, the second sensor electrodes are connected to the second bus electrode, and the first sensor electrodes and the second sensor electrodes are not connected.

The plurality of first sensor electrodes and the plurality of second sensor electrodes may be alternately arranged at intervals capable of detecting a change in electrical resistance of the sensitive film, and examples thereof include a pair of comb-tooth electrodes.

The interval between the first sensor electrodes and the second sensor electrodes may be an interval in which a change in the electrical resistance of the sensitive film can be detected. The interval may be, for example, 100 µm or more, and may be 500 µm or more. Also, the interval may be, for example, 10 mm or less, and may be 5 mm or less. That is, the interval may be, for example, 100 µm or more and 10 mm or less, and may be 500 µm or more and 5 mm or less.

The interval between the first sensor electrode and the second sensor electrode refers to the distance from the end portion of the first sensor electrode to the end portion of the second sensor electrode adjacent to each other. For example, in FIG. 9, the interval between the first sensor electrode and the second sensor electrode is indicated by d1.

The width of the first sensor electrode and the width of the second sensor electrode may be a width capable of detecting a change in the electrical resistance of the sensitive film. The width is, for example, 100 µm or more and may be 500 µm or more. Also, the width may be, for example, 10 mm or less, and may be 5 mm or less. That is, the width may be, for example, 100 µm or more and 10 mm or less, and may be 500 µm or more and 5 mm or less. For example, in FIG. 9, the width of the first sensor electrode is indicated by b1, and the width of the second sensor electrode is indicated by b2.

The length of the first sensor electrode and the length of the second sensor electrode may be a length capable of detecting a change in the electrical resistance of the sensitive film. The length is, for example, 10 mm or more and may be 50 mm or more. Also, the length may be, for example, 500 mm or less, and may be 100 mm or less. That is, the length may be, for example, 10 mm or more and 500 mm or less, and may be 50 mm or more and 100 mm or less. For example, in FIG. 9, the length of the first sensor electrode is indicated by a1, and the length of the second sensor electrode is indicated by a2.

The overlapping length of the first sensor electrode and the second sensor electrode may be any length capable of detecting a change in the electrical resistance of the sensitive film. The overlapping length is, for example, 9 mm or more and may be 45 mm or more. Also, the overlapping length may be, for example, 450 mm or less, and may be 90 mm or less. That is, the overlapping length may be, for example, 9 mm or more and 450 mm or less, and may be 45 mm or more and 90 mm or less. For example, in FIG. 9, the overlapping length of the first sensor electrode and the second sensor electrode is indicated by c.

The number of the first sensor electrodes and the number of the second sensor electrodes are appropriately set according to the size of the hydrogen sensor, the arrangement of the first bus electrode, the arrangement of the second bus electrode, and the like.

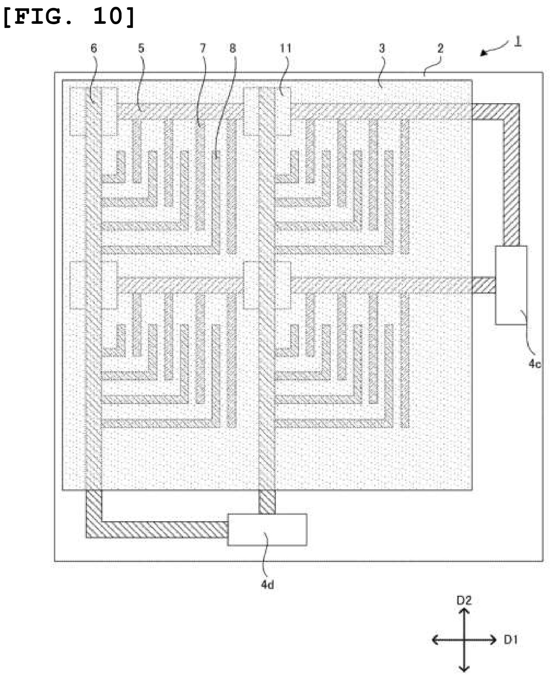

The shape of the first sensor electrode and the shape of the second sensor electrode are not particularly limited, and examples thereof include a linear shape, a polygonal line shape, and a curved line shape. Also, the shape of the first sensor electrode and the shape of the second sensor electrode may be a shape including a branch. For example, in FIG. 9, the shape of the first sensor electrode 7 is linear shape, and the shape of the second sensor electrode 8 is a shape including a branch. In addition, for example, in FIG. 10, the shape of the first sensor electrode 7 is linear shape, and the shape of the second sensor electrode 8 is a polygonal line shape.

The conductive material used for the first sensor electrodes and the second sensor electrodes, the thickness of the first sensor electrode and the second sensor electrode, and the method for forming the first sensor electrode and the second sensor electrode are the same as those of the first sensor electrodes and the second sensor electrodes in the first embodiment.

### 2. First bus electrode and second bus electrode

In the present embodiment, the first bus electrode and the second bus electrode are disposed on the first surface of the substrate and are connected to the control unit. The hydrogen sensor of the present embodiment includes a plurality of first bus electrodes and a plurality of second bus electrodes. The first bus electrodes are disposed along a first direction, and the second bus electrodes are disposed along a second direction orthogonal to the first direction.

The width of the first bus electrode and the width of the second bus electrode may be a width capable of functioning as an electrode. The width is, for example, 1 mm or more and may be 5 mm or more. Also, the width may be, for example, 50 mm or less, and may be 10 mm or less. That is, the width may be, for example, 1 mm or more and 50 mm or less, and may be 5 mm or more and 10 mm or less. For example, in FIG. 9, the width of the first bus electrode is indicated by e1, and the width of the second bus electrode is indicated by e2.

An interval between adjacent first bus electrodes and an interval between adjacent second bus electrodes may be an interval in which the first sensor electrodes and the second sensor electrodes can be disposed. The interval may be, for example, 11 mm or more, and may be 55 mm or more. Also, the interval may be, for example, 550 mm or less, and may be 110 mm or less. That is, the interval may be, for example, 11 mm or more and 550 mm or less, and may be 55 mm or more and 110 mm or less. For example, in FIG. 9, an interval between adjacent first bus electrodes is indicated by k1, and an interval between adjacent second bus electrodes is indicated by k2.

The number of the first bus electrodes and the number of the second bus electrodes are plural.

The conductive material used for the first bus electrode and the second bus electrode is the same as the conductive material used for the first sensor electrodes and the second sensor electrodes.

The thickness of the first bus electrode and the thickness of the second bus electrode are the same as the thickness of the first sensor electrode and the thickness of the second sensor electrode.

The method for forming the first bus electrode and the method for forming the second bus electrode are the same as the method for forming the first sensor electrode and the method for forming the second sensor electrode described above.

### 3. Sensitive film

The sensitive film in the present embodiment includes a catalyst dissociating hydrogen molecules, and tungsten oxide. The sensitive film is the same as the sensitive film in the first embodiment.

The sensitive film may be disposed in contact with the first sensor electrodes and the second sensor electrodes, and the position of the sensitive film is not particularly limited. For example, the first sensor electrodes and the second sensor electrodes, and the sensitive film may be arranged in this order on the first surface of the substrate; the sensitive film, and the first sensor electrodes and the second sensor electrodes may be arranged in this order on the first surface of the substrate; the first sensor electrodes, the sensitive film, and the second sensor electrodes may be arranged in this order on the first surface of the substrate; and the second sensor electrodes, the sensitive film, and the first sensor electrodes may be arranged in this order on the first surface of the substrate.

### 4. Control unit

The control unit in the present embodiment is a member that detects a change in electrical resistance. The first bus electrode and the second bus electrode are connected to the control unit.

The control unit is the same as the control unit in the first embodiment.

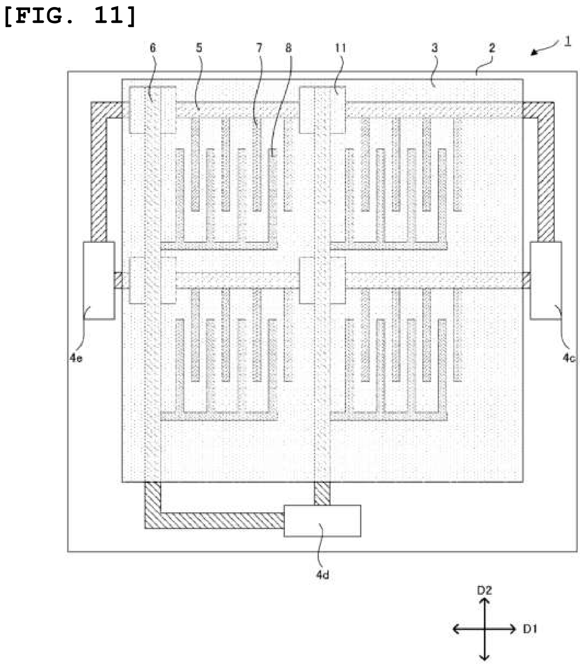

The control unit includes a third control unit connected to one end of the plurality of first bus electrodes, and a fourth control unit connected to one end of the plurality of second bus electrodes. In addition, the control unit may include the third control unit, the fourth control unit, and a fifth control unit connected to the other end of the plurality of first bus electrodes. In addition, the control unit may include the third control unit, the fourth control unit, and a sixth control unit connected to the other end of the plurality of second bus electrodes. In addition, the control unit may include the third control unit, the fourth control unit, the fifth control unit, and the sixth control unit. For example, in FIG. 11, the control unit includes a third control unit 4c connected to one end of the plurality of first bus electrodes 5, a fourth control unit 4d connected to one end of the plurality of second bus electrodes 6, and a fifth control unit 4e connected to the other end of the plurality of first bus electrodes 5.

### 5. Substrate

The substrate in the present embodiment is a member that supports the sensitive film, the first sensor electrodes, the second sensor electrodes, the first bus electrode, and the second bus electrode, and has insulating property. The substrate is the same as the substrate in the first embodiment.

### 6. Insulating film

The insulating film in the present embodiment is disposed between the first bus electrodes and the second bus electrodes in a region where the first bus electrodes and the second bus electrodes intersect.

The material of the insulating film is not particularly limited as long as it has insulating property, and examples thereof include an inorganic oxide, an inorganic nitride, an inorganic carbide, and a resin.

The thickness of the insulating film is not particularly limited as long as it can insulate the first bus electrode and the second bus electrode, and is, for example, 0.1 µm or more and 2 µm or less.

The method for forming the insulating film is not particularly limited, and examples thereof include a method for forming and patterning an insulating film, a mask vapor deposition method, and a printing method. Examples of the method for forming the insulating film include a vacuum vapor deposition method, a sputtering method, an ion plating method, and a plating method. Examples of the patterning method include an etching method and a lift-off method.

### B. Hydrogen detection system

In the hydrogen detection system in the present disclosure, the hydrogen sensor described above is used.

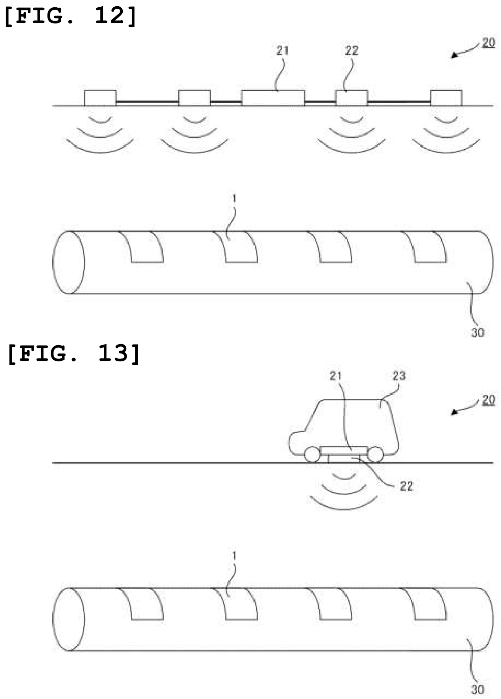

FIG. 12 is a schematic diagram illustrating an example of a hydrogen detection system in the present disclosure. In FIG. 12, the hydrogen detection system 20 includes a plurality of hydrogen sensors 1, an RFID reader/writer 21, and a plurality of antennas 22 connected to the RFID reader/writer 21. The hydrogen sensor 1 is attached to a hydrogen pipeline 30 installed underground. The RFID reader/writer 21 and the antennas 22 are installed at any place near the ground, and are fixed.

FIG. 13 is a schematic diagram illustrating another example of the hydrogen detection system in the present disclosure. In FIG. 12, the hydrogen detection system 20 includes a plurality of hydrogen sensors 1, an RFID reader/writer 21, and an antenna 22 connected to the RFID reader/writer 21. The hydrogen sensor 1 is attached to a hydrogen pipeline 30 installed underground. The RFID reader/writer 21 and the antenna 22 are installed in a moving body 23 and are movable.

In such a hydrogen detection system, the IC chip configuring the control unit of the hydrogen sensor is driven in a non-contact manner, and thereby a change in the electrical resistance of the sensitive film can be detected, and the hydrogen gas can be detected.

The hydrogen detection system in the present disclosure is not particularly limited as long as it is a system using a hydrogen sensor, but is preferably a system using an RFID. Specifically, the hydrogen detection system in the present disclosure includes a hydrogen sensor, an RFID reader/writer, and an antenna connected to the RFID reader/writer.

The RFID reader/writer may be of a fixed type or a movable type. When a fixed type RFID reader/writer is used, continuous monitoring is possible. On the other hand, when a movable RFID reader/writer is used, trace inspection can be performed, and the trace inspection can be advanced and smart, and labor-saving is possible.

The hydrogen detection system in the present disclosure can be used not only in a small device such as a fuel cell, but can also be used in a large facility such as a hydrogen production facility, a hydrogen pipeline, a transport tanker, a storage tank, and a hydrogen power generation facility.

### C. Sheet for hydrogen sensor

A sheet for hydrogen sensor in the present disclosure is a sheet hydrogen sensor in a roll shape, and includes: a substrate; a sensitive film that is disposed on a first surface of the substrate and that includes a catalyst dissociating hydrogen molecules, and tungsten oxide; a bus electrode disposed on the first surface of the substrate and disposed in a meandering line shape; and a plurality of first sensor electrodes and a plurality of second sensor electrodes disposed in contact with the sensitive film on the first surface of the substrate, and connected to the bus electrode, wherein the first sensor electrodes and the second sensor electrodes are alternately arranged at intervals in which a change in electrical resistance of the sensitive film can be detected.

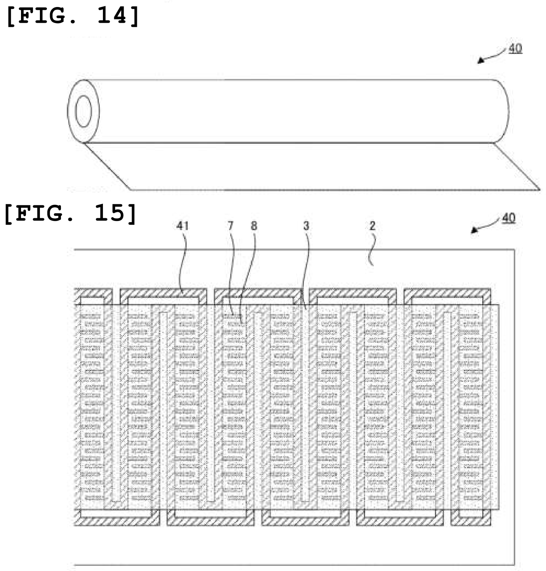

FIG. 14 is a schematic perspective view illustrating an example of a sheet for hydrogen sensor in the present disclosure, and FIG. 15 is a schematic plan view illustrating an example of a sheet for hydrogen sensor in the present disclosure. As shown in FIG. 14, the sheet for hydrogen sensor 40 is in a roll shape. As shown in FIG. 15, the sheet for hydrogen sensor 40 includes a substrate 2, a sensitive film 3 that is disposed on the first surface of the substrate 2 and that includes a catalyst dissociating hydrogen molecules, and tungsten oxide, a bus electrode 41 disposed on the first surface of the substrate 2 and arranged in a meandering line shape, and a plurality of first sensor electrodes 7 and a plurality of second sensor electrodes 8 disposed in contact with the sensitive film 3 on the first surface of the substrate 2, and connected to the bus electrode 41. The first sensor electrodes 7 and the second sensor electrodes 8 are alternately arranged at intervals in which a change in the electrical resistance of the sensitive film 3 can be detected.

The sheet for hydrogen sensor in the present disclosure is used for producing the hydrogen sensor described above. Since the sheet for hydrogen sensor in the present disclosure can be produced by a roll-to-roll method, the hydrogen sensor can be efficiently mass-produced.

The bus electrode, the first sensor electrodes, the second sensor electrodes, and the sensitive film in the sheet for hydrogen sensor of the present disclosure are the same as the first bus electrode, the second bus electrode, the first sensor electrodes, the second sensor electrodes, and the sensitive film in the first embodiment of the hydrogen sensor described above except that the first bus electrode and the second bus electrode are connected in the first embodiment of the hydrogen sensor.

In the case of producing the above-described hydrogen sensor using the sheet for hydrogen sensor in the present disclosure, the sheet for hydrogen sensor is cut according to the target length, and the bus electrode of the portion to which the control unit is connected is cut. After that, the control unit is disposed.

Incidentally, the present disclosure is not limited to the above-described embodiments. The above-described embodiments are examples, and those having substantially the same configuration as the technical idea described in the claims of the present disclosure and exhibiting similar operations and effects are included in the technical scope of the present disclosure.

### Examples

Hereinafter, examples will be described, and the present disclosure will be described in more detail.

The hydrogen sensor shown in FIG. 4 was produced. A first bus electrode, a second bus electrode, first sensor electrodes, and second sensor electrodes were formed on a 50 µm-thick polyethylene terephthalate (PET) film by a printing method. The length a1 of the first sensor electrode and the length a2 of the second sensor electrode were 70 mm, the width b1 of the first sensor electrode and the width b2 of the second sensor electrode were 2 mm, the overlapping length c of the first sensor electrodes and the second sensor electrodes was 50 mm, the interval d1 between the first sensor electrodes and the second sensor electrodes was 2 mm, the width e1 of the first bus electrode and the width e2 of the second bus electrode were 10 mm, the interval f between the first bus electrode and the second bus electrode facing each other across the first sensor electrodes and the second sensor electrodes was 90 mm, the interval g between the first bus electrode and the second bus electrode facing each other without sandwiching the first sensor electrodes and the second sensor electrodes was 10 mm, and the interval h1 between the adjacent first bus electrodes and the interval h2 between the adjacent second bus electrodes were 10 mm. Also, the thickness of the first bus electrode, the thickness of the second bus electrode, the thickness of the first sensor electrode, and the thickness of the second sensor electrode were 0.2 µm, respectively. Next, a sensitive film was formed on the PET film by a sol-gel method so as to cover the first bus electrode, the second bus electrode, the first sensor electrodes, and the second sensor electrodes. Next, the IC tag was mounted and connected to the first bus electrode and the second bus electrode. As a result, a hydrogen sensor was obtained.

It was confirmed that the radio wave transmitted by the RFID reader/writer was transmitted to the hydrogen sensor, and the radio wave to be reflected and transmitted was detected. In addition, when the radio wave was transmitted in the same manner as described above while the hydrogen sensor was exposed to hydrogen gas, the signal level of the radio wave reflected and transmitted was changed. As a result, it was confirmed that hydrogen gas was detected without mounting a power source on the hydrogen sensor itself.

The leakage position from the first control unit was specified by multiplying the total length of the first bus electrode or the second bus electrode by the coefficient obtained by dividing R1 by the total resistance value (R1 + R2) by using the resistance value R1 measured by the first control unit and the resistance value R2 measured by the second control unit. Although the resistance value changed depending on the concentration of hydrogen, the leakage position was accurately specified regardless of the hydrogen concentration by the above method.

In the present disclosure, the following inventions are provided.
[1] A hydrogen sensor comprising:
   a substrate,
   a sensitive film that is disposed on a first surface of the substrate, and that includes a catalyst dissociating hydrogen molecules, and tungsten oxide,
   a control unit that detects a change in electrical resistance,
   a first bus electrode and a second bus electrode disposed on the first surface of the substrate and connected to the control unit,
   a plurality of first sensor electrodes disposed in contact with the sensitive film on the first surface of the substrate and connected to the first bus electrode, and
   a plurality of second sensor electrodes disposed in contact with the sensitive film on the first surface of the substrate and connected to the second bus electrode, wherein
   the first sensor electrodes and the second sensor electrodes are alternately arranged at intervals in which a change in electrical resistance of the sensitive film can be detected.
[2] The hydrogen sensor according to [1], wherein one of the first bus electrode and one of the second bus electrode are each arranged in a line shape drawable with a one stroke, and
   one end of the first bus electrode and one end of the second bus electrode are connected to the control unit.
[3] The hydrogen sensor according to [2], wherein the control unit includes a first control unit connected to one end of the first bus electrode and one end of the second bus electrode, and a second control unit connected to the other end of the first bus electrode and the other end of the second bus electrode.
[4] The hydrogen sensor according to [2] or [3], wherein the first bus electrode and the second bus electrode are arranged in a meandering line shape.
[5] The hydrogen sensor according to [1], wherein a plurality of the first bus electrodes and a plurality of the second bus electrodes are connected to the control unit via a flexible printed circuit board.
[6] The hydrogen sensor according to [1], wherein
   a plurality of the first bus electrodes are arranged along a first direction,
   a plurality of the second bus electrodes are arranged along a second direction orthogonal to the first direction,
   the control unit includes a third control unit connected to one end of the plurality of the first bus electrodes, and a fourth control unit connected to one end of the plurality of the second bus electrodes, and
   an insulating film is disposed between the first bus electrodes and the second bus electrodes in a region where the first bus electrodes and the second bus electrodes intersect.
[7] A hydrogen detection system using the hydrogen sensor according to any one of [1] to [6].
[8] A sheet for hydrogen sensor in a roll shape, the sheet for hydrogen sensor comprising:
   a substrate,
   a sensitive film that is disposed on a first surface of the substrate and that includes a catalyst dissociating hydrogen molecules, and tungsten oxide,
   a bus electrode disposed on the first surface of the substrate and disposed in a meandering line shape, and
   a plurality of first sensor electrodes and a plurality of second sensor electrodes disposed in contact with the sensitive film on the first surface of the substrate, and connected to the bus electrode, wherein
   the first sensor electrodes and the second sensor electrodes are alternately arranged at intervals in which a change in electrical resistance of the sensitive film can be detected.

### Reference Signs List

- 1: hydrogen sensor
- 2: substrate
- 3: sensitive film
- 4: control unit
- 4a: first control unit
- 4b: second control unit
- 4c: third control unit
- 4d: fourth control unit
- 5: first bus electrode
- 6: second bus electrode
- 7: first sensor electrode
- 8: second sensor electrode
- 20: hydrogen detection system
- 40: sheet for hydrogen sensor

## Claims

1. A hydrogen sensor comprising:
a substrate,
a sensitive film that is disposed on a first surface of the substrate, and that includes a catalyst dissociating hydrogen molecules, and tungsten oxide,
a control unit that detects a change in electrical resistance,
a first bus electrode and a second bus electrode disposed on the first surface of the substrate and connected to the control unit,
a plurality of first sensor electrodes disposed in contact with the sensitive film on the first surface of the substrate and connected to the first bus electrode, and
a plurality of second sensor electrodes disposed in contact with the sensitive film on the first surface of the substrate and connected to the second bus electrode, wherein
the first sensor electrodes and the second sensor electrodes are alternately arranged at intervals in which a change in electrical resistance of the sensitive film can be detected.

2. The hydrogen sensor according to claim 1, wherein one of the first bus electrode and one of the second bus electrode are each arranged in a line shape drawable with a one stroke, and
one end of the first bus electrode and one end of the second bus electrode are connected to the control unit.

3. The hydrogen sensor according to claim 2, wherein the control unit includes a first control unit connected to one end of the first bus electrode and one end of the second bus electrode, and a second control unit connected to the other end of the first bus electrode and the other end of the second bus electrode.

4. The hydrogen sensor according to claim 2, wherein the first bus electrode and the second bus electrode are arranged in a meandering line shape.

5. The hydrogen sensor according to claim 1, wherein a plurality of the first bus electrodes and a plurality of the second bus electrodes are connected to the control unit via a flexible printed circuit board.

6. The hydrogen sensor according to claim 1, wherein
a plurality of the first bus electrodes are arranged along a first direction,
a plurality of the second bus electrodes are arranged along a second direction orthogonal to the first direction,
the control unit includes a third control unit connected to one end of the plurality of the first bus electrodes, and a fourth control unit connected to one end of the plurality of the second bus electrodes, and
an insulating film is disposed between the first bus electrodes and the second bus electrodes in a region where the first bus electrodes and the second bus electrodes intersect.

7. A hydrogen detection system using the hydrogen sensor according to any one of claim 1 to claim 6.

8. A sheet for hydrogen sensor in a roll shape, the sheet for hydrogen sensor comprising:
a substrate,
a sensitive film that is disposed on a first surface of the substrate and that includes a catalyst dissociating hydrogen molecules, and tungsten oxide,
a bus electrode disposed on the first surface of the substrate and disposed in a meandering line shape, and
a plurality of first sensor electrodes and a plurality of second sensor electrodes disposed in contact with the sensitive film on the first surface of the substrate, and connected to the bus electrode, wherein
the first sensor electrodes and the second sensor electrodes are alternately arranged at intervals in which a change in electrical resistance of the sensitive film can be detected.
